(19) Europäisches Patentamt
European Patent Office
Office européen des brevets

(11) Veröffentlichungsnummer : **0 085 347**
**B1**

(12) # EUROPÄISCHE PATENTSCHRIFT

(45) Veröffentlichungstag der Patentschrift : **18.09.85**

(51) Int. Cl.[4] : **C 07 C 69/96**, C 07 C 68/04

(21) Anmeldenummer : **83100384.3**

(22) Anmeldetag : **18.01.83**

(54) **Herstellung von Dialkylcarbonaten.**

(30) Priorität : **30.01.82 DE 3203190**
**29.10.82 DE 3240082**

(43) Veröffentlichungstag der Anmeldung :
**10.08.83 Patentblatt 83/32**

(45) Bekanntmachung des Hinweises auf die Patenterteilung : **18.09.85 Patentblatt 85/38**

(84) Benannte Vertragsstaaten :
**BE CH DE FR GB IT LI NL**

(56) Entgegenhaltungen :
**CHEMICAL ABSTRACTS, Band 90, Nr. 21, 21. Mai 1979, Seite 558, Nr. 168087r, Columbus, Ohio, USA**

(73) Patentinhaber : **BAYER AG**
**Konzernverwaltung RP Patentabteilung**
**D-5090 Leverkusen 1 Bayerwerk (DE)**

(72) Erfinder : **Genz, Joachim, Dipl.-Chem.**
**Alte Kasseler Strasse 2**
**D-3550 Marburg / Lahn (DE)**
Erfinder : **Heitz, Walter, Prof. Dr.**
**Am Schmidtborn 5**
**D-3575 Kirchhain 1 (DE)**

**EP 0 085 347 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, daß man Kohlendioxid und aliphatische Alkohole mit Hilfe von Zinndibutylalkoxiden als Katalysatoren unter Wasserabspaltung zu den entsprechenden Dialkylcarbonaten umsetzen kann. Die Reaktion verläuft gemäß

$$CO_2 + 2\ ROH \underset{}{\overset{\text{Katalysator}}{\rightleftharpoons}} RO-\overset{O}{\overset{\|}{C}}-OR + H_2O.$$

Die Ausbeuten dieser Reaktion sind gering, da das Veresterungsgleichgewicht stark auf der Seite der Ausgangsmaterialien liegt (siehe Yamazaki et al., Pol. Preprints, Amer. Chem. Soc., Div. Polym. Chem. 20 (1979) Seite 146 bis 149, bzw. Jap. Offenlegungsschrift vom 11.1.1979 Nr. 79/3012).

Gegenstand der Erfindung ist eine Verbesserung dieser Methode, die zu höheren Ausbeuten führt.

Gegenstand der Erfindung ist daher ein Verfahren zur Herstellung von Dialkylcarbonaten in Anwesenheit eines Katalysators, dadurch gekennzeichnet, daß man einen einwertigen aliphatischen Alkohol bei Temperaturen von 100 bis 220 °C und bei einem Druck von 1 bis 3 000 bar in Anwesenheit eines Molekularsiebs mit Kohlendioxid umsetzt, wobei als Katalysatoren Sn-Verbindungen der allgemeinen Formel

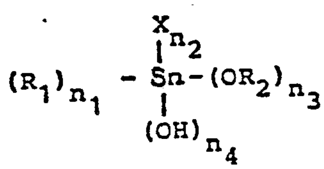

eingesetzt werden, worin

$R_1$ $C_1$-$C_{18}$ Alkyl oder $C_6$-$C_{20}$ Aryl ist,
$R_2$ $C_1$-$C_{18}$ Alkyl, $C_6$-$C_{20}$ Aryl oder $C_2$-$C_{18}$ Acyl ist,
X Halogen, SH, = O/2 oder = S/2 ist und
$n_1$, $n_2$, $n_3$ und $n_4$ ganze Zahlen von 0 bis 4 sind,

wobei die Summe aus $n_1 + n_2 + n_3 + n_4$ jeweils 4 ist.

Als Kohlendioxid verwendet man beispielsweise gasförmiges Kohlendioxid.

Im allgemeinen verwendet man für diese Umsetzung Autoklaven, in die der Alkohol, der Katalysator und das Molekularsieb gebracht werden, die dann mit beispielsweise gasförmigem Kohlendioxid bis zu dem gewünschten Druck gefüllt werden und anschließend auf die Reaktionstemperatur gebracht werden. Das gebildete Carbonat kann nach Entspannen und Öffnen des Autoklaven nach konventionellen Methoden isoliert werden.

Für das Verfahren geeignete einwertige aliphatische Alkohole sind lineare primäre Alkohole, verzweigte primäre Alkohole oder sekundäre Alkohole, insbesondere $C_1$-$C_{10}$-Alkanole wie Ethanol, Methanol, Propanol, Isopropanol, Butanol, Hexanol, 2-Ethylhexanol, 2-Methylpropanol, 3-Methylbutanol, Amylalkohol und Butanol-2.

Erfindungsgemäß geeignete Katalysatoren sind Verbindungen der 4. Hauptgruppe und leiten sich vom Element Zinn ab; Beispiele sind Orthostannate (z. B. Tetraphenyl-orthostannat) etc.

Geeignete Katalysatoren für das erfindungsgemäße Verfahren sind auch Kombinationen der vorstehend genannten Sn-Verbindungen, wobei Elektronendonator- und Elektronenakzeptor-Eigenschaften der Katalysatorkombination aufeinander abzustimmen sind, d.h. Kombinationen von Elektronendonatoren und Elektronenakzeptoren einzusetzen sind.

Die erfindungsgemäß geeigneten Katalysatoren werden in Mengen von $10^{-3}$ bis 5 Mol-%, vorzugsweise von 0,1 bis 3 Mol.-%, bezogen auf Mole an jeweils eingesetzten Alkohol eingesetzt, und zwar gilt dies sowohl für den Einsatz von einzelnen Katalysatoren als auch von Katalysatorkombinationen. Das molare Verhältnis der Katalysatorarten in den Katalysatorkombinationen kann zwischen 1 : 3 und 3 : 1 variieren und liegt beispielsweise bei 1 : 1.

Erfindungsgemäße Katalysatoren sind Sn-Verbindungen der allgemeinen Formel

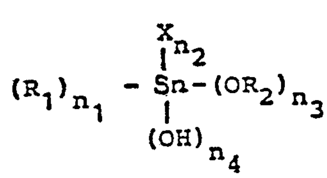

worin Me Sn ist,

$R_1$ $C_1$-$C_{18}$ Alkyl oder $C_6$-$C_{20}$ Aryl ist,
$R_2$ $C_1$-$C_{18}$ Alkyl, $C_6$-$C_{20}$ Aryl oder $C_2$-$C_{18}$ Acyl ist,

X Halogen, SH, = O/2 oder = S/2 ist und
$n_1$, $n_2$, $n_3$ und $n_4$ ganze Zahlen von 0 bis 4 sind,
wobei die Summe aus $n_1 + n_2 + n_3 + n_4$ jeweils 4 ist.

Insbesondere gilt für Me = Sn $n_1$ = 1 oder 2.

Besonders bevorzugte Katalysatoren sind beispielsweise Dialkylzinndialkoxide wie Dibutylzinndibutylat oder Dibutylzinndilaurat.

Die erfindungsgemäß zu verwendenden Molekularsiebe sind bekannt (siehe beispielsweise DE-OS 2 350 327, Spalte 3, Zeilen 28 bis 65 sowie Ullmanns Encyklopädie der technischen Chemie, 4. Auflage, Bd. 17, Seiten 9 bis 18, Verlag Chemie, Weinheim 1979).

Als Molekularsiebe kommen insbesondere Zeolithe in Frage, deren Kanäle Durchmesser von 3 bis 4 Å haben.

Das Verfahren kann mit oder ohne Lösungs- bzw. Verdünnungsmittel durchgeführt werden. Als Lösungs- oder Verdünnungsmittel sind alle gegenüber der Reaktion inerten Flüssigkeiten geeignet.

Für das Verfahren setzt man Kohlendioxiddrücke von 1 bar bis 3 000 bar, insbesondere von 1 bar bis 1 000 bar ein, bevorzugt eingesetzt werden 10 bar bis 500 bar und ganz besonders bevorzugt 25 bar bis 300 bar.

Im allgemeinen arbeitet man bei Temperaturen von 0 bis 300 °C. Bevorzugt liegen die Reaktionstemperaturen allerdings zwischen 100 und 220 °C, besonders bevorzugte Temperaturen liegen zwischen 130 und 180 °C.

Die Reaktionszeiten können zwischen 2 und 100 Stunden, insbesondere zwischen 10 und 60 Stunden und insbesondere zwischen 24 und 48 Stunden liegen.

Die für das erfindungsgemäße Verfahren einzusetzende Menge an Molekularsieb muß jeweils so bemessen sein, daß das bei der Reaktion abgespaltene Wasser gebunden werden kann. Es kann auch etwas mehr oder etwas weniger als die für die Wasserbindung erforderliche Menge Molekularsieb eingesetzt werden.

Eine besondere Verfahrensvariante besteht darin, daß man die Umsetzung in mehreren Etappen, vorzugsweise bis zu fünf Etappen durchführt, wobei nach jeder Etappe die Reaktionslösung von dem Molekularsieb abgetrennt und mit frischem Molekularsieb versetzt wird.

Die in den nachfolgenden Beispielen angegebenen Ausbeuten an Carbonat beziehen sich jeweils auf die eingesetzten Mengen an Alkohol.

### Beispiel 1

Ein aliphatischer Alkohol (25 g 2-Ethylhexanol = 193,5 mMol) wurde mit einem Katalysator (3,3 g Dibutylzinndilaurat = 5,5 mMol ~ 3 Mol-%) und einer wasserentziehenden Substanz (50 g Molekularsieb 4 Å) in einen 200 ml fassenden Laborautoklaven gegeben. Durch mehrmaliges Aufpressen von Kohlendioxid mit anschließendem Entlüften wurde die enthaltene Luft entfernt. Anschließend wurden 55 bar $CO_2$ aufgepreßt, zur Durchmischung mehrmals umgeschüttelt und der Autoklav für 66 Stunden auf 160 °C temperiert. Danach wurde der Autoklav abkühlen lassen, überschüssiges $CO_2$ abgelassen und der Anteil an entstandenem Carbonat gaschromatographisch ermittelt. Der Anteil an Di-(2-Ethylhexyl)-carbonat betrug 22,2 % (Flächen-%), bezogen auf eingesetzten Alkohol (wie auch in den nachfolgenden Beispielen).

### Beispiel 2

Eingesetzte Mengen wie unter Beispiel 1. Es wurde ein 250 ml fassender Laborautoklav verwendet. Die Reaktionstemperatur betrug 220 °C, die Reaktionszeit 18 Stunden. Die Ausbeute betrug 20,2 %.

### Beispiel 3

Es wurden 1,25 g 2-Ethylhexanol (= 9,6 mMol), 0,083 g Dibutylzinndilaurat (= 0,14 mMol ~ 1,5 mMol-%) und 2,5 g Molekularsieb 3 Å eingesetzt. Verwendet wurde ein 10 ml fassender Hochdruckautoklav, auf den 3 000 bar $CO_2$ aufgepreßt wurden. Die Reaktionstemperatur betrug 200 °C, die Reaktionszeit 42 Stunden. Die Ausbeute betrug 16,6 %.

### Beispiel 4

Eingesetzte Mengen wie unter Beispiel 1. Es wurde ein 200 ml fassender Laborautoklav verwendet. Nach jeweils 18 Stunden Reaktionszeit bei 160 °C wurde die Reaktion unterbrochen die Reaktionslösung vom Molekularsieb abgetrennt, mit frischem Molekularsieb versetzt und erneut $CO_2$ aufgepreßt. Nach 4 Reaktionscyclen betrug die Ausbeute an Carbonat 60,1 %.

### Beispiel 5 (Vergleichsbeispiel)

12,5 g 2-Ethylhexanol (= 96 mMol) und 2,7 g Dibutylzinndilaurat (= 4,5 mMol = 4,7 Mol-%) wurden mit 50 ml Xylol und 10,3 g N,N'-Dicyclohexylcarbodiimid (= 50 mMol) in einem 250 ml Laborautoklaven

eingesetzt. Die Reaktionslösung wurde unter 45 bar $CO_2$-Druck 66 Stunden bei Raumtemperatur gerührt. Die Ausbeute an Carbonat betrug 8,1 %.

Beispiel 6 (Vergleichsbeispiel)

Eingesetzte Mengen wie unter Beispiel 5. Es wurde ein 200 ml fassender Laborautoklav verwendet. Die Reaktionstemperatur betrug 80 °C, die Reaktionszeit 66 Stunden. Es wurden 4 % Carbonat erhalten.

Beispiel 7 (Vergleichsbeispiel)

Eingesetzte Mengen wie unter Beispiel 5. Reaktion in 200 ml Laborautoklaven. Reaktionstemperatur 120 °C, Reaktionszeit 9 Stunden. Ausbeute an Carbonat 7,3 %.

Beispiel 8

Es wurden 40,45 g n-Butanol (= 545,65 mMol), 1.61 g Dibutyldimethoxyzinn (= 5,46 mMol = 1 Mol%) und 50 g Molekularsieb 3 A in einem 200 ml fassenden Laborautoklaven mit 55 bar $CO_2$ versetzt. Die Reaktionszeit betrug 91 h, die Reaktionstemperatur 155 °C. Die Ausbeute an Di(n-butyl)-carbonat betrug 22,0 % (Flächen%).

Beispiel 9

Es wurden 10.0 g n-Heptanol (= 86,05 mMol) und 1.27 g Dibutyldimethoxyzinn (= 4,30 mMol = 5 Mol%) mit 50 ml Xylol und 50 g Molekularsieb im 250 ml Autoklav mit 55 bar $CO_2$ versetzt. Die Reaktionstemperatur betrug 155 °C, die Reaktionszeit 50 h. Die Ausbeute an Di(n-heptyl) carbonat betrug 19.4 % (Flächen%).

**Patentansprüche**

1. Verfahren zur Herstellung von Dialkylcarbonaten in Anwesenheit eines Katalysators, dadurch gekennzeichnet, daß man einen einwertigen aliphatischen Alkohol bei Temperaturen von 100 bis 220 °C und bei einem Druck von 1 bis 3 000 bar in Anwesenheit eines Molekularsiebs mit Kohlendioxid umsetzt, wobei als Katalysatoren Sn-Verbindungen der allgemeinen Formel

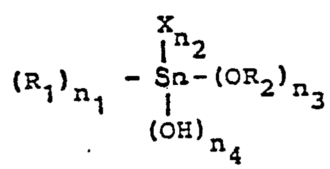

eingesetzt werden, worin

$R_1$ $C_1$-$C_{18}$ Alkyl oder $C_6$-$C_{20}$ Aryl ist,

$R_2$ $C_1$-$C_{18}$ Alkyl, $C_6$-$C_{20}$ Aryl oder $C_2$-$C_{18}$ Acyl ist,

X Halogen, SH, = O/2 oder = S/2 ist und

$n_1$, $n_2$, $n_3$ und $n_4$ ganze Zahlen von 0 bis 4 sind,

wobei die Summe aus $n_1 + n_2 + n_3 + n_4$ jeweils 4 ist.

2. Verfahren gemäß Anspruch 1, dadurch gekennzeichnet, daß man als Molekularsiebe Zeolithe einsetzt.

3. Verfahren gemäß Ansprüche 1 und 2, dadurch gekennzeichnet, daß man die Umsetzung in mehreren Etappen durchführt.

4. Verfahren gemäß Ansprüche 1 bis 3, dadurch gekennzeichnet, daß man bei Drücken von 1 bis 1 000 bar die Umsetzung durchführt.

5. Verfahren gemäß Anspruch 4, dadurch gekennzeichnet, daß man bei Drücken von 10 bis 500 bar die Umsetzung durchführt.

6. Verfahren gemäß Ansprüche 1 bis 5, dadurch gekennzeichnet, daß man bei Temperaturen von 130 bis 180 °C die Umsetzung durchführt.

7. Verfahren gemäß Anspruch 1, dadurch gekennzeichnet, daß als Katalysatoren der allgemeinen Formel solche verwendet werden, worin $n_1$ = 1 oder 2 ist.

**Claims**

1. Process for the preparation of dialkyl carbonates in the presence of a catalyst, characterised in that a monohydric aliphatic alcohol is reacted with carbon dioxide at temperatures of 100 to 220 °C and

under a pressure of 1 to 3,000 bar in the presence of a molecular sieve, Sn compounds of the general formula

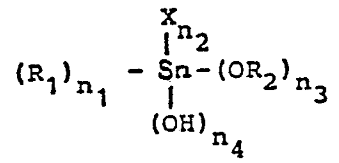

wherein

$R_1$ is $C_1$-$C_{18}$-alkyl or $C_6$-$C_{20}$-aryl,

$R_2$ is $C_1$-$C_{18}$-alkyl, $C_6$-$C_{20}$-aryl or $C_2$-$C_{18}$-acyl,

X is halogen, SH, = O/2 or = S/2 and

$n_1$, $n_2$, $n_3$ and $n_4$ are integers from 0 to 4,

the sum of $n_1 + n_2 + n_3 + n_4$ being in each case 4, being used as catalysts.

2. Process according to Claim 1, characterised in that zeolites are used as molecular sieves.

3. Process according to Claims 1 and 2, characterised in that the reaction is carried out in several stages.

4. Process according to Claims 1 to 3, characterised in that the reaction is carried out under pressures of 1 to 1,000 bar.

5. Process according to Claim 4, characterised in that the reaction is carried out under pressures of 10 to 500 bar.

6. Process according to Claims 1 to 5, characterised in that the reaction is carried out at temperatures of 130 to 180 °C.

7. Process according to Claim 1, characterised in that the catalysts of the general formula used are those in which $n_1$ = 1 or 2.

**Revendications**

1. Procédé de production de carbonates de dialkyle en présence d'un catalyseur, caractérisé en ce qu'on fait réagir un alcool aliphatique monovalent avec l'anhydride carbonique à des températures de 100 à 220 °C et à une pression de 1 à 3 000 bars en présence d'un tamis moléculaire, en utilisant comme catalyseurs des composés d'étain de formule générale

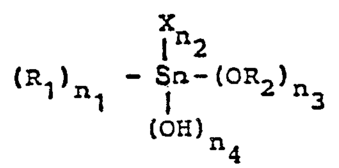

dans laquelle

$R_1$ est un groupe alkyle en $C_1$ à $C_{18}$ ou aryle en $C_6$ à $C_{20}$,

$R_2$ est un groupe alkyle en $C_1$ à $C_{18}$, aryle en $C_6$ à $C_{20}$ ou acyle en $C_2$ à $C_{18}$,

X est un halogène, SH, = O/2 ou = S/2 et

$n_1$, $n_2$, $n_3$ et $n_4$ sont des nombres entiers de 0 à 4,

la somme $n_1 + n_2 + n_3 + n_4$ étant dans chaque cas égale à 4.

2. Procédé suivant la revendication 1, caractérisé en ce qu'on utilise des zéolites comme tamis moléculaires.

3. Procédé suivant les revendications 1 et 2, caractérisé en ce qu'on conduit la réaction en plusieurs étapes.

4. Procédé suivant les revendications 1 à 3, caractérisé en ce qu'on conduit la réaction à des pressions de 1 à 1 000 bars.

5. Procédé suivant la revendication 4, caractérisé en ce qu'on conduit la réaction à des pressions de 10 à 500 bars.

6. Procédé suivant les revendications 1 à 5, caractérisé en ce qu'on conduit la réaction à des températures de 130 à 180 °C.

7. Procédé suivant la revendication 1, caractérisé en ce qu'on utilise comme catalyseurs de la formule générale ceux dans lesquels $n_1$ est égal à 1 ou 2.